# EUROPEAN PATENT APPLICATION

(11) **EP 4 470 560 A1**
(43) Date of publication of application: **04.12.2024**
(21) Application number: 22923150.1
(22) Date of filing: 17.06.2022
(51) Int. Cl.: A61K 39/39, A61K 47/64, A61K 38/08, C07K 14/77, A61P 35/00

(54) **TUMOR IMMUNOTHERAPY**

(30) Priority: 26.01.2022 CN 202210093765
(71) Applicant: Pingshan Translational Medicine Center, Shenzhen Bay Laboratory, Shenzhen, Guangdong 518118 (CN); Peking University Shenzhen Graduate School, Shenzhen, Guangdong 518055 (CN)
(72) Inventor: LI, Zigang, Shenzhen, Guangdong 518118 (CN); YIN, Feng, Shenzhen, Guangdong 518118 (CN); ZHANG, Yaping, Shenzhen, Guangdong 518118 (CN); JIANG, Leying, Shenzhen, Guangdong 518118 (CN); LIU, Zhaodi, Shenzhen, Guangdong 518118 (CN)
(74) Representative: Müller-Boré & Partner Patentanwälte PartG mbB
(86) International application number: PCT/CN2022/099481
(87) International publication number: WO 2023/142341

(57) **Abstract**

Disclosed is a composition comprising a closed-loop peptide comprising at least two methionines and at least two sulfonium centers; and a conjugate of a negatively charged immunoadjuvant and a peptide.

## Description

### FIELD

The present disclosure generally relates to the field of medicine, and more particularly, to the field of tumor immunotherapy.

### BACKGROUND

Tumor immunotherapy has been known as the third revolution in cancer treatment. Unlike surgical resection, chemotherapy and radiotherapy, which directly act on the tumor site, tumor immunotherapy generates a specific immune response (e.g., activation of killer T cells) by activating the host immune system, indirectly targets the tumor cells accurately and can form a long-term memory, thereby effectively preventing recurrence of the tumor.

Neoantigens are produced by somatic mutations and are expressed only in tumor cells, effectively avoiding host central thymic tolerance limitations, with high tumor specificity and immunogenicity. Therefore, targeting them can induce the host immune system to circumvent normal cells accurately, making it possible to individualize accurate immunotherapy of tumors.

### SUMMARY

In one aspect, the present disclosure relates to a composition, comprising a closed-loop peptide comprising at least two methionines and at least two sulfonium centers; and a conjugate formed by a negatively charged immunoadjuvant and a peptide.

In another aspect, the present disclosure relates to a process for preparing a composition, comprising dissolving a linear peptide comprising at least two methionines in a weakly acidic polar solution and adding a closed-loop reagent to obtain a closed-loop peptide; mixing an immunoadjuvant with a sulfonium peptide under basic conditions to obtain the conjugate by an addition reaction; and mixing the conjugate with the closed-loop peptide to obtain the composition, wherein the composition comprises a closed-loop peptide comprising at least two methionines and at least two sulfonium centers; and a conjugate formed by a negatively charged immunoadjuvant and a peptide.

In a further aspect, the present disclosure relates to a method for treating or preventing a tumor, comprising administering to a subject in need thereof a therapeutically or prophylactically effective amount of a composition, wherein the composition comprises a closed-loop peptide comprising at least two methionines and at least two sulfonium centers; and a conjugate formed by a negatively charged immunoadjuvant and a peptide.

In yet another aspect, the present disclosure relates to a method for activating immunity, comprising administering to a subject in need thereof an effective amount of a composition, wherein the composition comprises a closed-loop peptide comprising at least two methionines and at least two sulfonium centers; and a conjugate formed by a negatively charged immunoadjuvant and a peptide.

In another aspect, the present disclosure relates to a nanoparticle, comprising: a closed-loop peptide comprising at least two methionines and at least two sulfonium centers; and a conjugate formed by a negatively charged immunoadjuvant and a peptide, wherein the closed-loop peptide is self-assembled with the conjugate to form the nanoparticle.

In a further aspect, the present disclosure relates to a composition for treating or preventing a tumor, comprising a closed-loop peptide comprising at least two methionines and at least two sulfonium centers; and a conjugate formed by a negatively charged immunoadjuvant and a peptide.

In yet another aspect, the present disclosure relates to a composition for activating immunity, comprising a closed-loop peptide comprising at least two methionines and at least two sulfonium centers; and a conjugate formed by a negatively charged immunoadjuvant and a peptide.

In another aspect, the present disclosure relates to use of a composition in preparation of a medicament for treating or preventing a tumor, wherein the composition comprises a closed-loop peptide comprising at least two methionines and at least two sulfonium centers; and a conjugate formed by a negatively charged immunoadjuvant and a peptide.

In a further aspect, the present disclosure relates to use of a composition in preparation of a medicament for activating immunity, wherein the composition comprises a closed-loop peptide comprising at least two methionines and at least two sulfonium centers; and a conjugate formed by a negatively charged immunoadjuvant and a peptide.

### BRIEF DESCRIPTION OF THE DRAWINGS

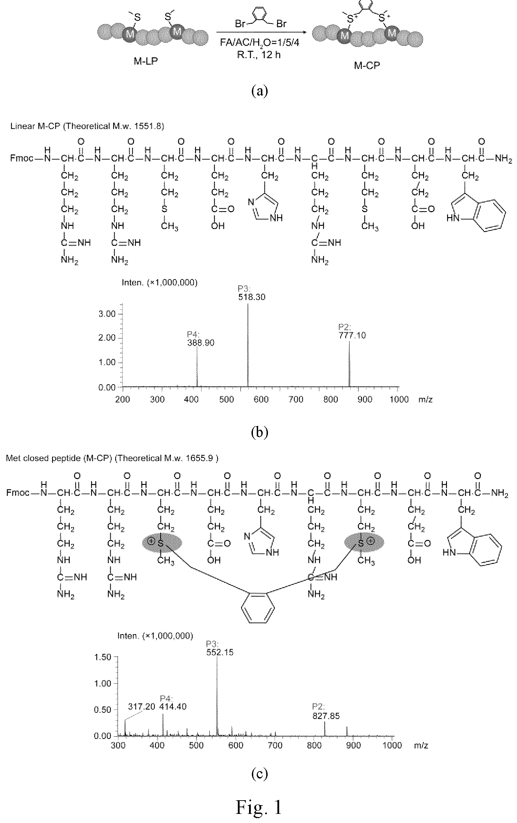

Fig. 1 shows the preparation and mass spectrometric identification results of the M-CP closed-loop peptide , wherein (a) shows a schematic diagram of the preparation of the M-CP closed-loop peptide; (b) shows the structure and mass spectrometry results of a linear M-CP peptide, wherein P2, P3, P4 represent the positions of peaks in the mass spectrometry when the peptides are carried with 2, 3 and 4 electrons, respectively; and (c) shows the structure and mass spectrum results of the peptide after closure (peptide sequence information: M-CP: Fmoc-RRMEHRMEW, SEQ ID NO 1).

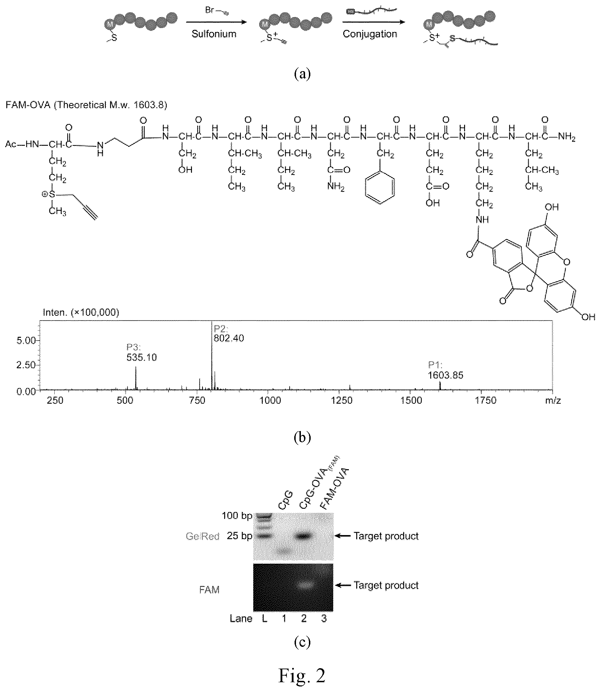

Fig. 2 shows the results of covalent conjugation of CpG adjuvant and antigen peptide OVA, wherein (a) shows a schematic diagram of covalent conjugation of CpG and OVA; (b) shows the structure and MS identification results of FAM-OVA attached with bromopropyne; and (c) shows the electrophoresis verification results of covalent conjugation (peptide sequence information: FAM-OVA: Ac-WMβASIINFEK (FAM) L, SEQ ID NO 3).

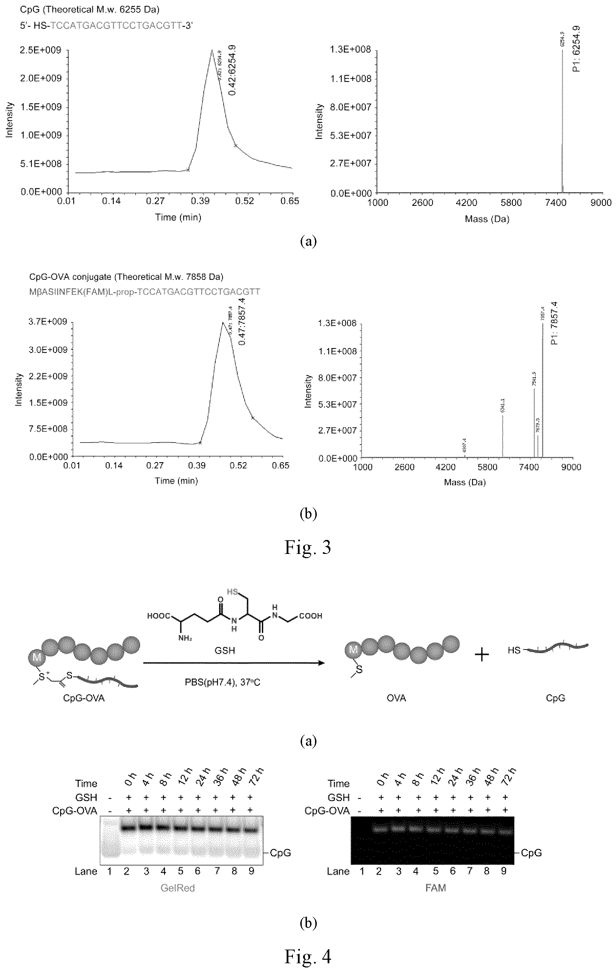

Fig. 3 shows the LC-MS identification results for covalent conjugation of CpG adjuvant and antigen peptide OVA, wherein (a) shows the LC-MS identification results of CpG; and (b) shows the LC-MS identification results of the CpG-OVA covalent conjugate (peptide sequence information: FAM-OVA: Ac-MβASIINFEK(FAM)L, SEQ ID NO 3; sequence information of CpG: 5'-3', HS-TCCATGACGTTCCTGACGTT, SEQ ID NO 6).
Fig. 4 shows the verification results of reversible reduction of CpG-OVA covalent conjugate by GSH, wherein (a) shows reduction of CpG-OVA by GSH; and (b) shows an electrophoretic verification map of the reduced product.

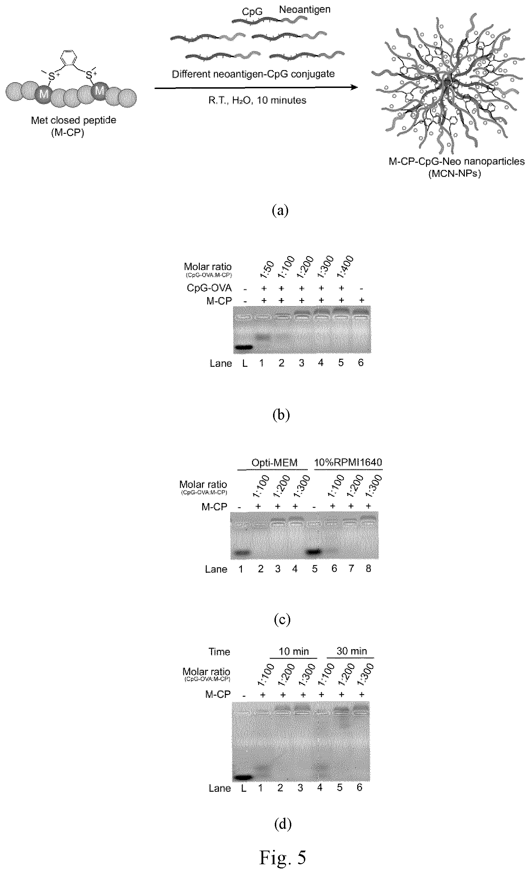

Fig. 5 shows the electrophoresis results of self-assembly (referred to as MCN-NPs) of M-CP closed-loop peptide induced by CpG-OVA conjugate, wherein (a) shows the self-assembly of M-CP closed-loop peptide induced by CpG-OVA conjugate; (b) shows the electrophoresis results of composite at different substrate ratios; (c) shows the self-assembly results of the composite under different buffer solution conditions; and (d) shows the electrophoresis results of nanocomposites prepared at different mixing times.

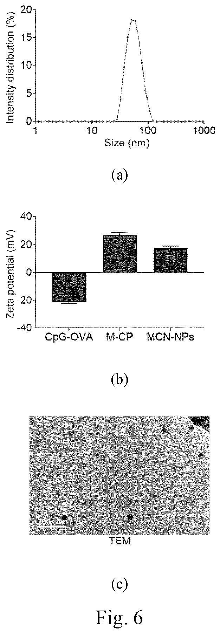

Fig. 6 shows the DLS analysis results for MCN-NPs, wherein (a) shows the particle size distribution of the nanocomposite; (b) shows a potential variation diagram of the nanocomposite; and (c) shows the TEM electron microscope results of the nanocomposite.

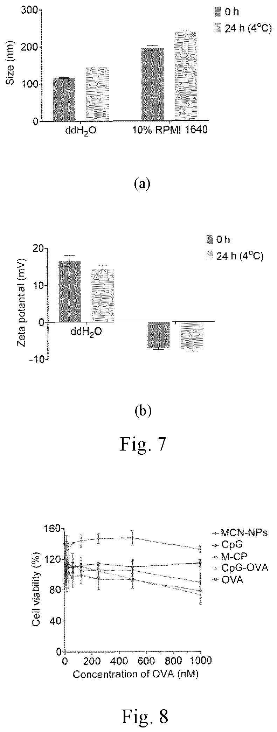

Fig. 7 shows the stability analysis results of MCN-NPs in different buffers, wherein (a) shows the statistics of the particle size change; and (b) shows the statistics of the potential change.
Fig. 8 shows the results of the inhibition of RAW264.7 cell growth by MCN-NPs based on MTT assay analysis (in terms of the concentration of OVA).

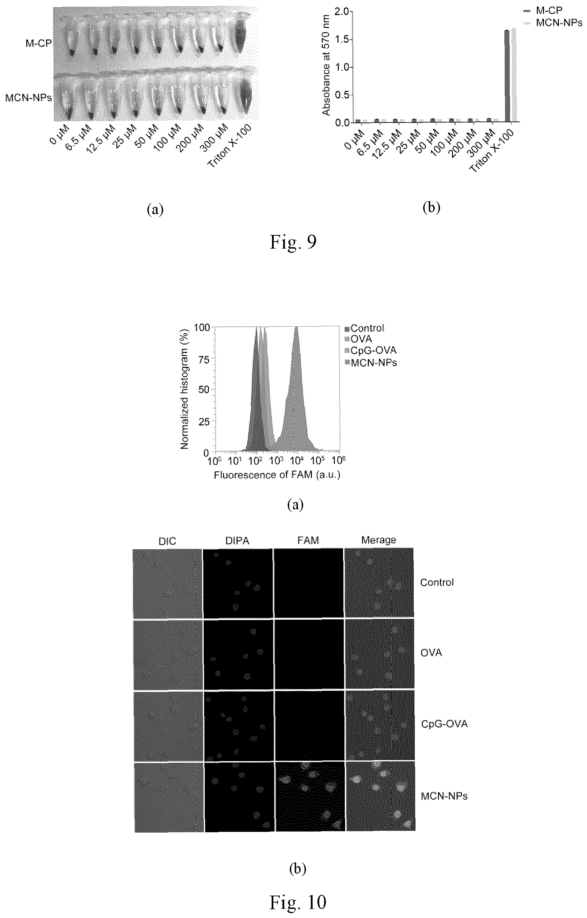

Fig. 9 shows the hemolytic assessment results of MCN-NPs (measured in M-CP), wherein (a) shows a picture of the supernatant after centrifugation; and (b) shows the absorbance of the supernatant at 570 nm.
Fig. 10 shows the assessment results of the transmembrane ability of MCN-NPs, wherein (a) shows the statistical results of the amount of fluorescence of MCN-NPs in RAW264.7 cells 4 hours after administration; and (b) shows the fluorescence localization of MCN-NPs in RAW264.7 cells 4 h after administration.

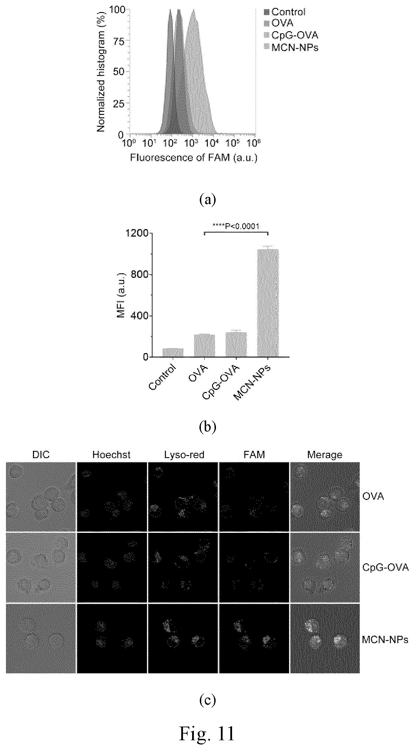

Fig. 11 shows the statistical results of the fluorescence amounts of MCN-NPs in DC2.4 cells, wherein (a) shows the statistical results of the fluorescence amounts of MCN-NPs in DC2.4 cells 4 hours after administration; (b) shows the median axis value statistics of the amount of fluorescence of MCN-NPs in DC2.4 cells 4 hours after administration; and (c) shows the fluorescence localization of MCN-NPs in DC2.4 cells 4 hours after administration.

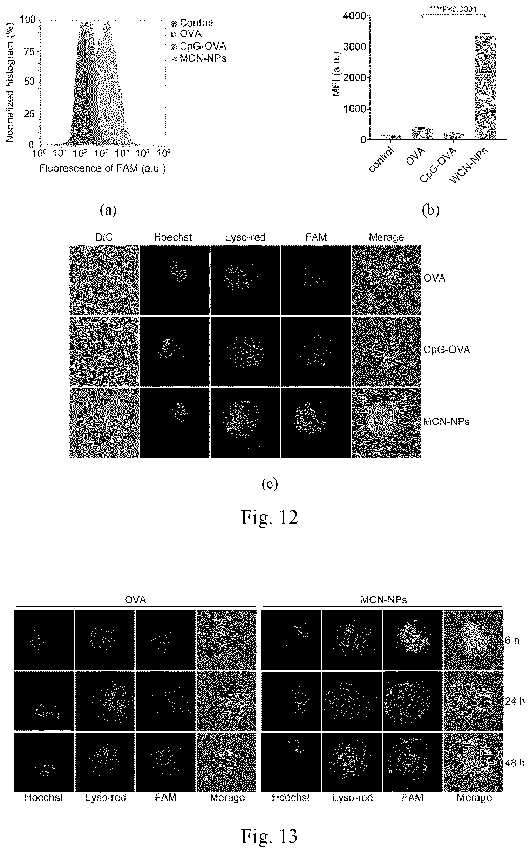

Fig. 12 shows the fluorescence quantitative analysis results of MCN-NPs in BMDCs cells, wherein (a) shows the statistical results of the fluorescence amounts of MCN-NPs in BMDCs cells 4 hours after administration; (b) shows the median axis value statistics of the amount of MCN-NPs fluorescence in BMDCs cells 4 hours after administration; and (c) shows the distribution of MCN-NPs in BMDCs cells 4 hours after administration.
Fig. 13 shows the results of uptake of MCN-NPs by BMDCs cells at different time periods.

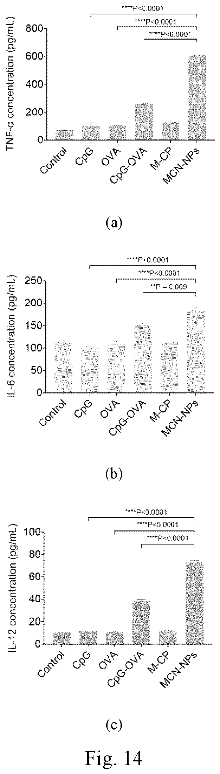

Fig. 14 shows the secretion of tumor-associated factors TNF-α., interleukin IL-6 and interleukin IL-12 by RAW264.7 cells stimulated by MCN-NPs, wherein (a) shows the ELISA analysis results of the secretion of tumor-associated factors TNF-α by RAW264.7 cells; (b) shows an ELISA analysis results of the secretion of interleukin IL-6 by RAW264.7 cells; and (c) shows the ELISA analysis results of interleukin IL-12 secretion by RAW264.7 cells.

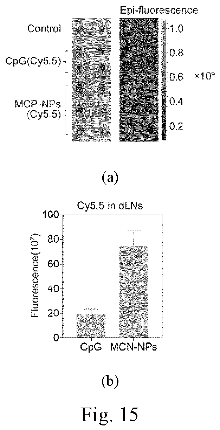

Fig. 15 shows the enrichment of MCN-NPs in lymph nodes, wherein (a) shows the size comparison of the lymph nodes and fluorescence imaging of Cy5.5 in lymph nodes 24 hours after subcutaneous administration; and (b) shows the statistics of the fluorescence intensity for Cy5.5.

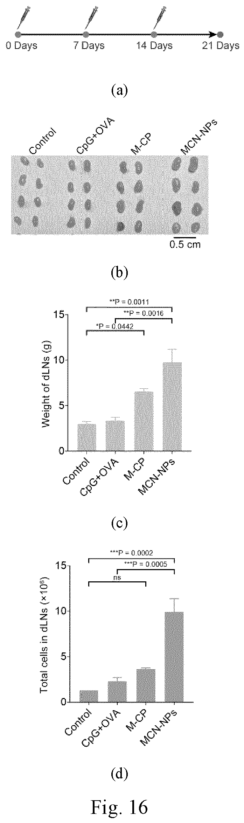

Fig. 16 shows the apparent assessment results of lymph nodes after three doses of immunization, wherein (a) shows the immunization dosing regimen; (b) shows the size comparison of the lymph nodes in each group of mice on day 7 after the last administration of MCN-NPs; (c) shows the statistics of the lymph node weight corresponding to (b); and (d) shows the total number of cells in the lymph nodes corresponding to (b).

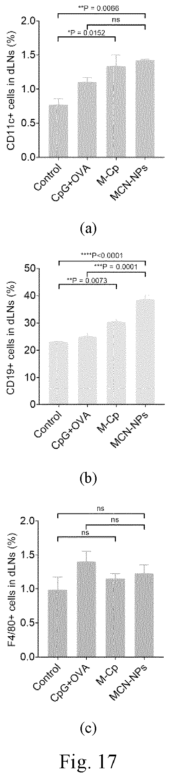

Fig. 17 shows the proliferation of each immune cell in the lymph node after three doses of immunization, wherein (a) shows the proportion of the DC cell population in the lymph node; (b) shows the proportion of the B cell population in the lymph node; and (c) shows the proportion of macrophages in the lymph nodes.

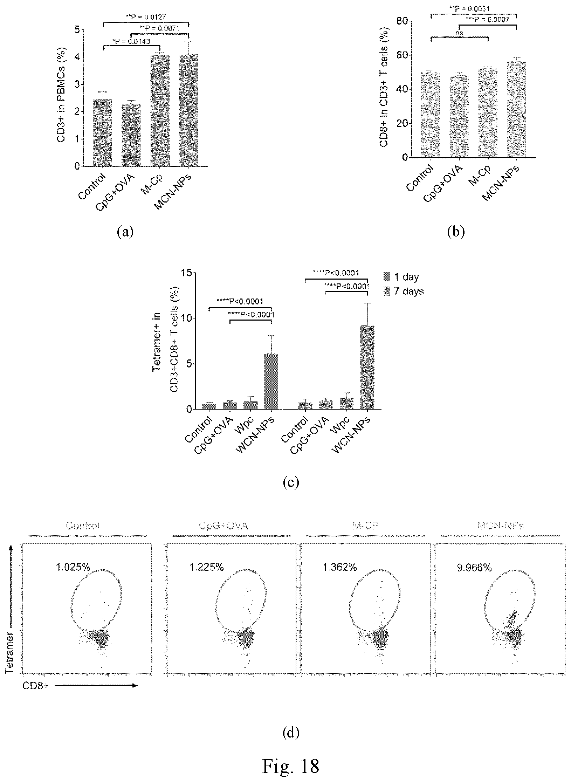

Fig. 18 shows the change of T cell population in the blood of mice after three doses of immunization, wherein (a) shows the change of the proportion of the total T cell population in the blood seven days after the last immunization; (b) shows the change of the proportion of CD8+T cells in the blood seven days after the last immunization; (c) shows the percentage of OVA-specific cells in CD8+T cells one day and seven days after the last immunization; and (d) shows the zoning results of OVA+CD8+T cells in the blood of each group of mice seven days after the last immunization.

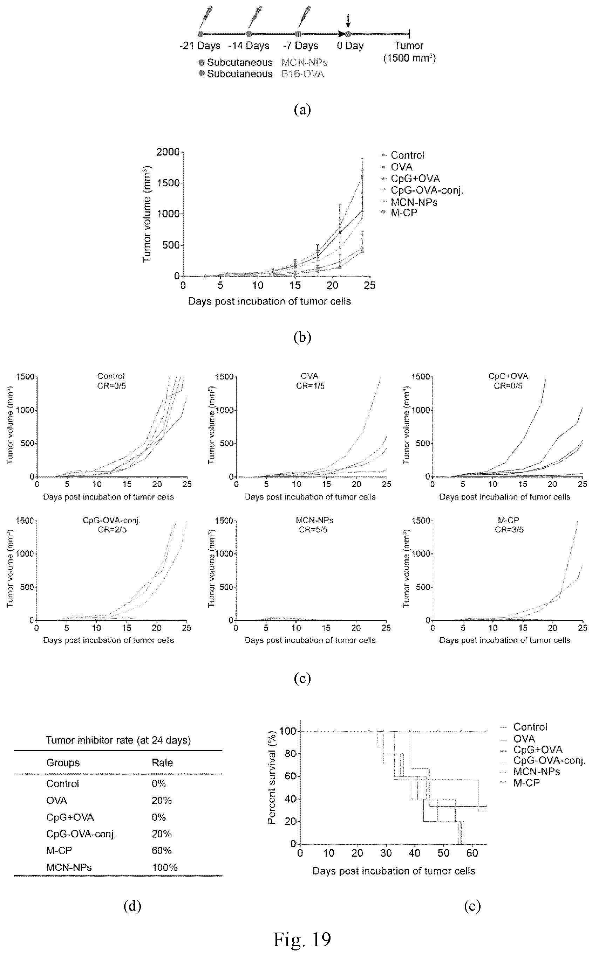

Fig. 19 shows the inhibition of the growth of the B16-OVA melanoma subcutaneously seeded after administration, wherein (a) shows the dosing regimen for the prevention of tumor; (b) shows the statistical data of tumor volume in mice within 24 days after seeding the tumor; (c) shows the monitoring of tumor growth in individual mice per group; (d) shows the effective inhibition rate of tumors in mice at 24 days after seeding; and (e) shows the statistics of the life cycle of mice.

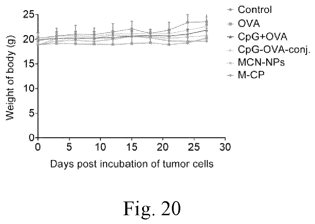

Fig. 20 shows the change of body weight in mice with B16-OVA melanoma subcutaneously seeded after administration.

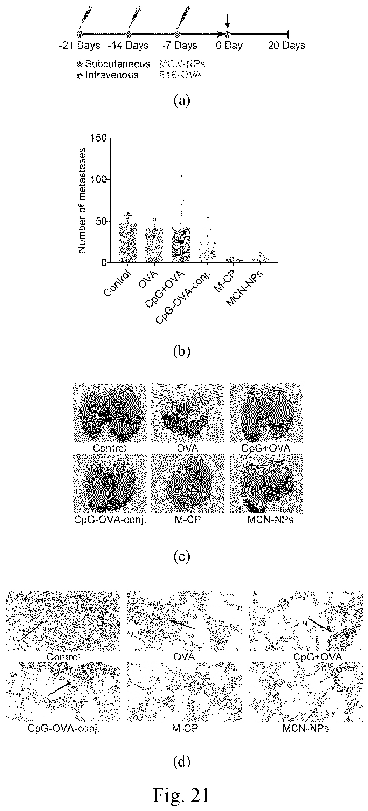

Fig. 21 shows the lung metastasis of tumor cells 20 days after B16-OVA melanoma is injected via tail vein after administration, wherein (a) shows the dosing regimen for the lung metastasis model; (b) shows the statistical data of the lung nodules; (c) shows the pictures of the mouse lungs; and (d) shows the H&E staining results of mouse lung sections.

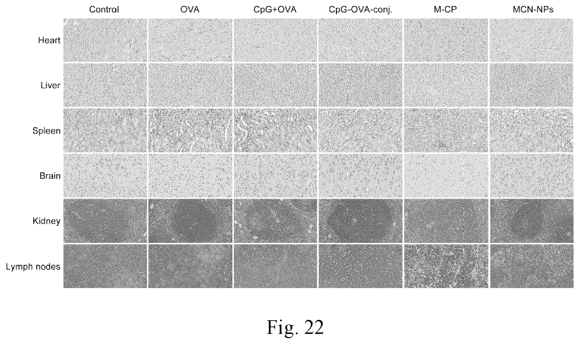

Fig. 22 shows the H&E staining of major tissue organs of mice 20 days after B16-OVA melanoma is injected via tail vein after administration.

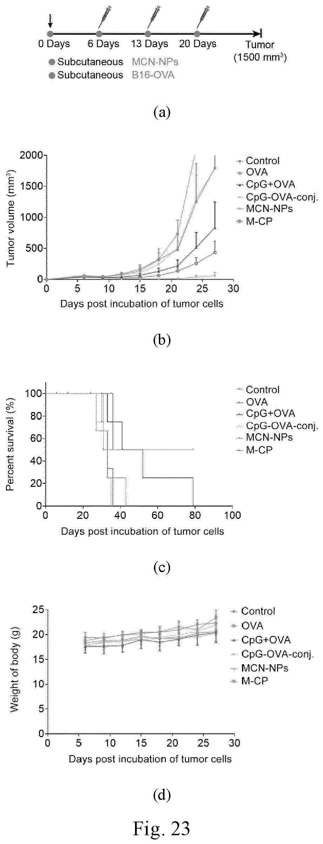

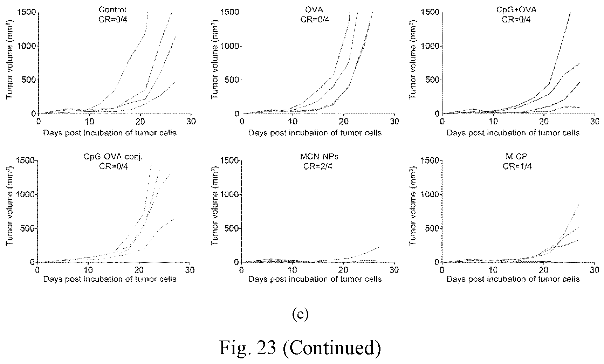

Fig. 23 shows the inhibition of tumor growth of B16-OVA seeded prior to administration, wherein (a) shows the dosing regimen for the tumor treatment model; (b) shows the statistical data of the tumor volume growth; (c) shows the statistics of the mouse life cycle; (d) shows the statistics of the mouse body weight; and (e) shows the monitoring of individual tumor growth in each group of mice.

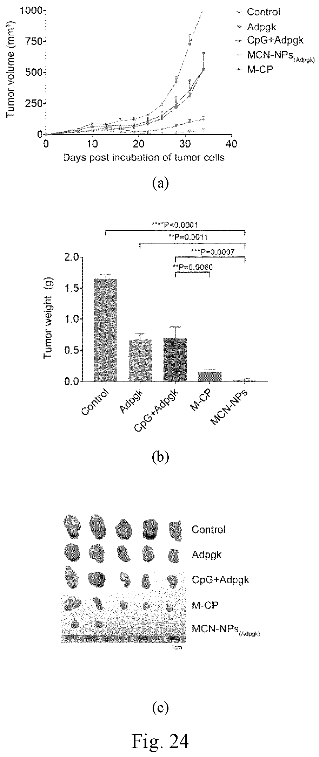

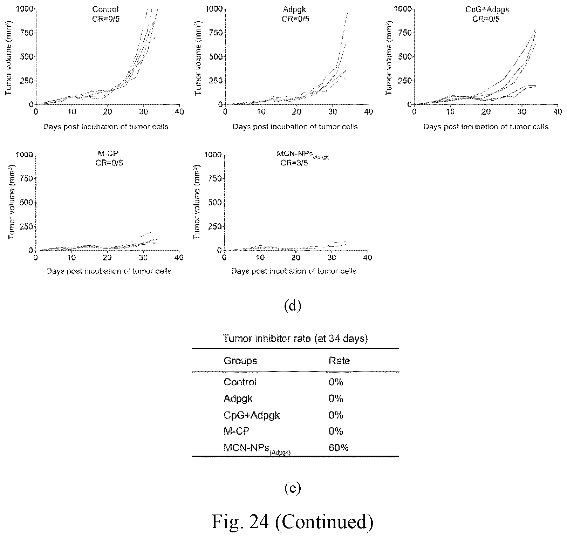

Fig. 24 shows the inhibition of MC-38 tumor growth by MCN-NPs_{(Adpgk)}, wherein (a) shows the growth curve of the tumor; (b) shows the weight statistics for each group of tumors on day 34; (c) shows the comparison plots of each group of tumors at day 34; (d) shows the growth curves of individual tumor of each group of mice; and (e) shows the tumor inhibition rate of each group of mice (peptide sequence information: Adpgk: Ac-ELASMTNMELMSS, SEQ ID NO 4).

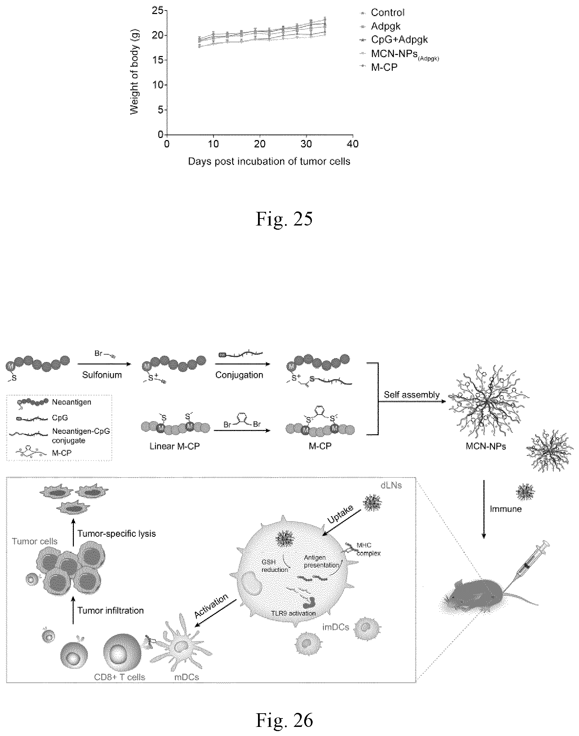

Fig. 25 shows the change in weight of mice in each group (MC-38 tumor group).
Fig. 26 shows a schematic diagram of the present disclosure.

### DETAILED DESCRIPTION

In the following description, certain specific details are included to provide a thorough understanding of the various disclosed embodiments. However, one skilled in the art will recognize that embodiments may be carried out without one or more of these specific details, but with other methods, components, materials and the like.

Unless otherwise claimed in this application, throughout the specification and the appended claims, the terms "comprise", "comprising," "contain" and "having" are to be construed in an open, inclusive sense, i.e., "including, but not limited to".

As used in this disclosure and the appended claims, the singular reference without an indication of quantity includes the plural reference unless the context clearly dictates otherwise.

Reference throughout this specification to "one embodiment" or "an embodiment" or "in another embodiment", or "in some embodiments" means that a particular referent feature structure or characteristic described in connection with the embodiments is included in at least one embodiment. Therefore, the appearance of the phrases "in one embodiment", "in the embodiment", "in another embodiment", or "in some embodiments" in various places throughout this specification are not necessarily all referring to the same embodiment. Moreover, the particular features structures or characteristics can be combined in any suitable manner in one or more embodiments.

It should be noted that, as used in this specification and the appended claims, the singular forms "a", "an" and "the" comprise plural referents unless the context clearly stated otherwise. Therefore, for example, a mentioned composition comprising "a closed-loop peptide comprising at least two methionines and at least two sulfonium centers" comprises one closed-loop peptide comprising at least two methionines and at least two sulfonium centers, or two or more closed-loop peptides comprising at least two methionines and at least two sulfonium centers.

### DEFINITION

As used herein, the term "sulfonium" refers to a compound comprising a tetravalent sulfur.

As used herein, the term "conjugate" refers to a novel compound formed by two different types of compounds via covalent linkage.

As used herein, the term "room temperature" refers to a temperature of 25°C at standard atmospheric pressure.

As used herein, the term "M-CP" refers to SEQ ID NO 1, of which the sequence (N→C) is Fmoc-RRMEHRMEW.

As used herein, the term "OVA" refers to SEQ ID NO 2, of which the sequence (N→C) is Ac-WMβASIINFEKL.

As used herein, the term "FAM-OVA" refers to SEQ ID NO 3, of which the sequence (N→C) is Ac-MβASIINFEK(FAM)L.

As used herein, the term "Adpgk" refers to SEQ ID NO 4, of which the sequence is Ac-ELASMTNMELMSS.

As used herein, the term "CpG" refers to SEQ ID NO 6, of which the sequence (5'-3') is HS-TCCATGACGTTCCTGACGTT.

As used herein, the term "Ac" refers to an amide group.

As used herein, the term "βA" refers to an isomer of alanine (A).

As used herein, the term "mammal" refers to animals including, for example, dogs, cats, cattle, sheep, horses, humans and the like. In some embodiments, mammal includes humans.

As used herein, the term "patient" refers to animals (e.g., humans), companion animals (e.g., dogs, cats or horses), and livestock (e.g., cattle, pigs and sheep). In some embodiments, the patient is a mammal comprising males and females. In some embodiments, the patient is a human.

"Treating" or "treatment" as used herein covers the treatment of the disease or condition of interest in a mammal, such as a human, having the disease or disorder of interest, and includes:
(i) preventing the disease or condition from occurring in a mammal, in particular, when such mammal is predisposed to the condition but has not yet been diagnosed as having it;
(ii) inhibiting the disease or condition, i.e., arresting its development; or
(iii) relieving the disease or condition, i.e., causing regression of the disease or condition.

### SPECIFIC EMBODIMENTS

In one aspect, the present disclosure relates to a composition, comprising a closed-loop peptide comprising at least two methionines and at least two sulfonium centers; and a conjugate formed by a negatively charged immunoadjuvant and a peptide.

In some embodiments, exemplary examples of a negatively charged immunoadjuvant that can be used in the present disclosure include, but are not limited to, a nucleic acid immune adjuvant, an activator of an immune pathway and an adjuvant having an effect of blocking an immunosuppressive pathway.

In some embodiments, exemplary examples of a negatively charged immunoadjuvant that can be used in the present disclosure include, but are not limited to, CpG₁₈₂₆, ploy(I:C) and siRNA/shRNA for silencing an immunosuppressive pathway or activating an immune pathway.

In some embodiments, a negatively charged immunoadjuvant that can be used in the present disclosure comprises at least 20 nucleotides.

In some embodiments, exemplary examples of a negatively charged immunoadjuvant that can be used in the present disclosure include, but are not limited to, a nucleic acid immunoadjuvant comprising a sequence of SEQ ID NO 6.

In some embodiments, exemplary examples of a negatively charged immunoadjuvant that can be used in the present disclosure include, but are not limited to, a nucleic acid immunoadjuvant shown by a sequence of SEQ ID NO 6.

In some embodiments, exemplary examples of a peptide that can be used in the present disclosure include, but are not limited to, an antigen peptide.

In some embodiments, exemplary examples of an antigen peptide that can be used in the present disclosure include, but are not limited to, an antigen peptide of melanoma B16-OVA (OVA257-264: Ac-SIINFEKL) and a neoantigen peptide of MC-38 tumor cells (Adpgk: Ac-ELASMTNMELMSS).

In some embodiments, exemplary examples of a peptide that can be used in the present disclosure include, but are not limited to, sulfonium peptides.

In some embodiments, exemplary examples of a peptide that can be used in the present disclosure include, but are not limited to, a peptide comprising a sequence of SEQ ID NO 2, a peptide comprising a sequence of SEQ ID NO 3, and a peptide comprising a sequence of SEQ ID NO 4.

In some embodiments, exemplary examples of a peptide that can be used in the present disclosure include, but are not limited to, a peptide shown by a sequence of SEQ ID NO 2, a peptide shown by a sequence of SEQ ID NO 3 and a peptide shown by a sequence of SEQ ID NO 4.

In some embodiments, exemplary examples of a peptide that can be used in the present disclosure include, but are not limited to, a closed-loop peptide comprising at least 9 amino acids.

In some embodiments, exemplary examples of a closed-loop peptide that can be used in the present disclosure include, but are not limited to, a closed-loop peptide comprising a sequence of SEQ ID NO 1.

In some embodiments, the structure of a closed-loop peptide that can be used in the present disclosure is as follows:

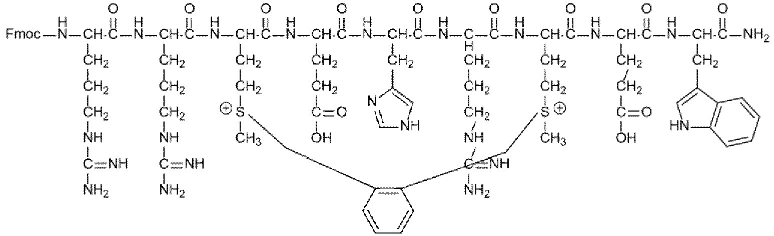

In some embodiments, exemplary examples of a conjugate that can be used in the present disclosure include, but are not limited to, a reversible covalent conjugate.

In some embodiments, the particle size of the composition is about 50 nm to 200 nm.

In some embodiments, the co-delivery system of the present disclosure is capable of rapid self-assembly and may simultaneously encompass immunoadjuvants, neoplastic neoantigens and neoplastic associated antigens, which solves the issues of long sequence, high cost and limited stability of a peptide-based nanoparticle support.

In another aspect, the present disclosure relates to a process for preparing a composition, comprising dissolving a linear peptide comprising at least two methionines in a weakly acidic polar solution and adding a closed-loop reagent to obtain a closed-loop peptide; mixing an immunoadjuvant with a sulfonium peptide under basic conditions to obtain a conjugate by an addition reaction; and mixing the conjugate with the closed-loop peptide to obtain the composition, wherein the composition comprises a closed-loop peptide comprising at least two methionines and at least two sulfonium centers; and a conjugate formed by a negatively charged immunoadjuvant and a peptide.

In some embodiments, exemplary examples of an immunoadjuvant that can be used in the process of the present disclosure include, but are not limited to, a nucleic acid adjuvant, an activator of an immune pathway and an adjuvant having an effect of blocking an immunosuppressive pathway.

In some embodiments, exemplary examples of an immunoadjuvant that can be used in the process of the present disclosure include, but are not limited to, an immunoadjuvant with a sulfhydryl terminus.

In some embodiments, exemplary examples of an immunoadjuvant that can be used in the process of the present disclosure include, but are not limited to, an immunoadjuvant comprising a sequence of SEQ ID NO 6.

In some embodiments, exemplary examples of an immunoadjuvant that can be used in the process of the present disclosure include, but are not limited to, an immunoadjuvant shown by a sequence of SEQ ID NO 6.

In some embodiments, exemplary examples of a sulfonium peptide that can be used in the process of the present disclosure include, but are not limited to, a sulfonium peptide having an alkynyl terminus and a sulfonium peptide having an alkenyl terminus.

In some embodiments, exemplary examples of a sulfonium peptide that can be used in the process of the present disclosure include, but are not limited to, a sulfonium peptide comprising a sequence of SEQ ID NO 2, a sulfonium peptide comprising a sequence of SEQ ID NO 3 and a sulfonium peptide comprising a sequence of SEQ ID NO 4.

In some embodiments, exemplary examples of a sulfonium peptide that can be used in the process of the present disclosure include, but are not limited to, a sulfonium peptide shown by a sequence of SEQ ID NO 2, a sulfonium peptide shown by a sequence of SEQ ID NO 3 and a sulfonium peptide shown by a sequence of SEQ ID NO 4.

In some embodiments, the molar ratio of an immunoadjuvant to a sulfonium peptide is about 1: 2 to 1: 10.

In some embodiments, the molar ratio of an immunoadjuvant to a closed-loop peptide is about 1:50 to 1:400.

In some embodiments, exemplary examples of a linear peptide comprising at least two methionines that can be used in the process of the present disclosure include, but are not limited to, a sequence of SEQ ID NO 1, a peptide comprising a sequence of SEQ ID NO 1, a sequence of SEQ ID NO 1 with one or more amino acid mutations and a peptide comprising a sequence of SEQ ID NO 1 with one or more amino acid mutations.

In some embodiments, exemplary examples of a linear peptide comprising at least two methionines that can be used in the process of the present disclosure include, but are not limited to, a linear peptide shown by a sequence of SEQ ID NO 1.

In some embodiments, a linear peptide comprising at least two methionines that can be used in the process of the present disclosure has the following structure:

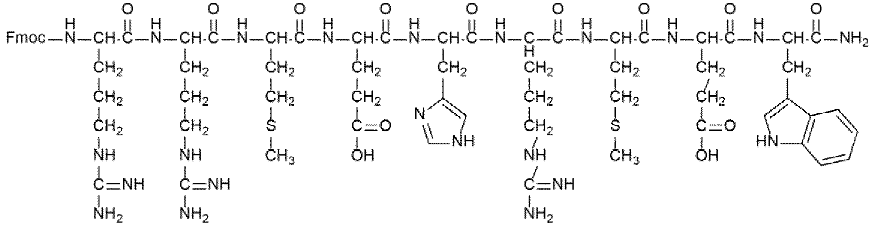

In some embodiments, exemplary examples of a weakly acidic polar solution that can be used in the process of the present disclosure include, but are not limited to, a mixing solution of formic acid/acetonitrile/water.

In some embodiments, the volume ratio of formic acid, acetonitrile and water in the mixing solution of formic acid/acetonitrile/water that can be used in the process of the present disclosure is about 1: (1 to 5): (1 to 4).

In some embodiments, exemplary examples of a closed-loop reagent that can be used in the present disclosure include, but are not limited to, o-dibromobenzyl, 1,3-bis(bromomethyl)benzene, 1,4-bis(bromomethyl) benzene, 1,4-dibromobutene, 2,3-bis(bromomethyl)quinoxaline, and 4,4'-dibromomethylbiphenyl.

In some embodiments, the concentration of a closed-loop reagent is about 6 mg/mL to 8 mg/mL.

In some embodiments, a conjugate is self-assembled with a closed-loop peptide to obtain a composition.

In some embodiments, an immunoadjuvant is mixed with a sulfonium peptide under basic conditions at about 4 to 28°C to obtain a conjugate by an addition reaction.

In some embodiments, an immunoadjuvant is mixed with a sulfonium peptide under basic conditions at room temperature to obtain a conjugate by an addition reaction.

In some embodiments, a linear peptide comprising at least two methionines is dissolved in a mixing solution of formic acid/acetonitrile/water and a closed-loop reagent is added and reacted for at least 8 hours to obtain a closed-loop peptide.

In some embodiments, the process of the present disclosure is simple and efficient.

In a further aspect, the present disclosure relates to a method for treating or preventing a tumor, comprising administering to a subject in need thereof a therapeutically or prophylactically effective amount of a composition, wherein the composition comprises a closed-loop peptide comprising at least two methionines and at least two sulfonium centers; and a conjugate formed by a negatively charged immunoadjuvant and a peptide.

In some embodiments, exemplary examples of a tumor that can be used in the present disclosure include, but are not limited to, melanoma and colon cancer.

In some embodiments, exemplary examples of a subject that can be used in the present disclosure include, but are not limited to, a mammal.

In some embodiments, exemplary examples of a subject that can be used in the present disclosure include, but are not limited to, a human.

In some embodiments, the method of the present disclosure for treating or preventing a tumor further comprises administering to the subject another agent for preventing or treating a tumor.

In some embodiments, exemplary examples of an agent for preventing or treating a tumor that can be used in the present disclosure include, but are not limited to, a chemotherapeutic agent, a biological drug and any combination thereof that can be administered in combination.

In some embodiments, exemplary examples of a chemotherapeutic agent for preventing or treating a tumor that can be used in the present disclosure include, but are not limited to, a small molecule chemotherapeutic agents

In some embodiments, exemplary examples of a small molecule chemotherapeutic agent for preventing or treating a tumor that can be used in the present disclosure include, but are not limited to, paclitaxel.

In some embodiments, exemplary examples of a biological drug for preventing or treating a tumor that can be used in the present disclosure include, but are not limited to, a neoplastic neoantigen peptide vaccine, a peptide drug and an antibody drug.

In some embodiments, exemplary examples of an antibody drug for preventing or treating a tumor that can be used in the present disclosure include, but are not limited to, PD-1 monoclonal antibody and PD-L1 monoclonal antibody.

In some embodiments, exemplary examples of PD-1 monoclonal antibody for preventing or treating a tumor that can be used in the present disclosure include, but are not limited to, nivolumab, pembrolizumab and tislelizumab.

In some embodiments, exemplary examples of PD-L1 monoclonal antibody for preventing or treating a tumor that can be used in the present disclosure include, but are not limited to, atezolizumab, durvalumab and avelumab.

In yet another aspect, the present disclosure relates to a method for activating immunity, comprising administering to a subject in need thereof an effective amount of a composition, wherein the composition comprises a closed-loop peptide comprising at least two methionines and at least two sulfonium centers; and a conjugate formed by a negatively charged immunoadjuvant and a peptide.

In some embodiments, the immunity is a tumor immunity.

In some embodiments, the immunity is a cellular immunity.

In another aspect, the present disclosure relates to a nanoparticle, comprising a closed-loop peptide comprising at least two methionines and at least two sulfonium centers; and a conjugate formed by a negatively charged immunoadjuvant and a peptide, wherein the closed-loop peptide is self-assembled with the conjugate to form the nanoparticle.

In certain embodiments, the nanoparticle of the present disclosure has a particle size of about 50 nm to 200 nm.

In a further aspect, the present disclosure relates to a composition for treating or preventing a tumor, comprising a closed-loop peptide comprising at least two methionines and at least two sulfonium centers; and a conjugate formed by a negatively charged immunoadjuvant and a peptide.

In yet another aspect, the present disclosure relates to a composition for activating immunity, comprising a closed-loop peptide comprising at least two methionines and at least two sulfonium centers; and a conjugate formed by a negatively charged immunoadjuvant and a peptide.

In another aspect, the present disclosure relates to use of a composition in preparation of a medicament for treating or preventing a tumor, wherein the composition comprises a closed-loop peptide comprising at least two methionines and at least two sulfonium centers; and a conjugate formed by a negatively charged immunoadjuvant and a peptide.

In a further aspect, the present disclosure relates to use of a composition in preparation of a medicament for activating immunity, wherein the composition comprises a closed-loop peptide comprising at least two methionines and at least two sulfonium centers; and a conjugate formed by a negatively charged immunoadjuvant and a peptide.

The present disclosure will hereinafter be explained in detail in order to better understand the aspects of the present application and its advantages. However, it is to be understood that the following examples are non-limiting and merely illustrative of certain embodiments of the present disclosure.

### Examples

The reagents and devices used in the embodiments of the present disclosure are both conventional and commercially available. For example:

| Name | CAS number | Manufacturer |
|---|---|---|
| Tribromopropyne | 106-96-7 | Energy Chemical |
| O-dibromobenzyl | 626-15-3 | Energy Chemical |
| MBHA resin | 431041-83-7 | Tianjin Nankaihe Chengke Technology Co., Ltd. |
| Dichloromethane (DCM) | 75-09-2 | Xilong Science |
| N,N-dimethylformamide (DMF) | 68-12-2 | Xilong Science |
| Morpholine | 110-91-8 | Aladdin |
| 6-chlorobenzotriazole-1,1,3,3-tetramethyluronium hexafluorophosphate (HCTU) | 330645-87-9 | Jier (Shanghai) Biochemistry Co., Ltd. |
| N,N-diisopropylethylamine (DIPEA) | 7087-68-5 | Energy Chemical |
| Trifluoroacetic acid (TFA) | 76-05-1 | Energy Chemical |
| Triisopropylsilane (TIPS) | 6485-79-6 | Energy Chemical |
| Acetic anhydride | 108-24-7 | Xilong Science |
| HS-CpG | | Suzhou Genewiz Biotechnology Co., Ltd. |
| TCEP | 115-96-8 | Merck Sigma-Aldrich |
| Sodium acetate | 6131-90-4 | Merck Sigma-Aldrich |
| Glacial ethanol | 64-17-5 | Xilong Science |
| GSH | 70-18-8 | Merck Sigma-Aldrich |
| MTT (3-(4,5-dimethylthiazol-2)-2,5-diphenyltetrazolium bromide) | 298-93-1 | Beijing Solarbio Technology Co., Ltd. |
| RAW 264.7 | | ATCC: The Global |
| | | Bioresource Center |
| DC2.4 | | ATCC: The Global Bioresource Center |
| C57 BL/6 | | Zhejiang Weitong Lihua Experimental Animal Technology Co., Ltd. |
| BMDCs | | Self-made |
| Cell flow buffer | S1001 | Multi Sciences (Lianke) Biotech Co., Ltd |
| 10* erythrocyte lysate | 70-LYS01 | Multi Sciences (Lianke) Biotech Co., Ltd |
| CD16/CD32 | 101320 | Biolegend |
| CD11c-APC | 17-4888-81 | Thermo Fisher Scientific |
| CD19-PE | 82168S | Cell Signaling Technology (CST) |
| F4/80-APC | 17-4801-82 | Thermo Fisher Scientific |
| CD3e-efluor450 | 48-0031-82 | Thermo Fisher Scientific |
| CD4-APC | 17-0042-82 | Thermo Fisher Scientific |
| CD8-FITC | 11-0081-82 | Thermo Fisher Scientific |
| MHC-OVA-APC | | Beijing Fubo Biotechnology Co., Ltd. |
| B16-OVA | | Liang Xingjie Group, National Nanoscience Center |
| MC-38 | | ATCC: The Global Bioresource Center |
| Paraformaldehyde | 30525-89-4 | Beyotime |

### Example 1

### Synthesis, Isolation and Purification of Linear Peptides

The desired peptide was obtained using conventional peptide solid phase synthesis methods.

**Table 1. Information of Peptide Sequence Involved in the Present Disclosure**

| Peptide | Sequence (N→C) | Length | |
|---|---|---|---|
| M-CP | Fmoc-RRMEHRMEW | 9 | SEQ ID NO 1 |
| OVA | Ac-WMβASIINFEKL | 11 | SEQ ID NO 2 |
| FAM-OVA | Ac-MβASIINFEK (FAM) L | 10 | SEQ ID NO 3 |
| Adpgk | Ac-ELASMTNMELMSS | 13 | SEQ ID NO 4 |

| | | | |
|---|---|---|---|
| Note: The terminal Fmoc of the M-CP peptide was not removed. AC refers to an amide group. βA refers to an isomer of alanine (A), commonly used as a spacer amino acid. The functional peptide segments (Ac-SIINFEKL, SEQ ID NO 5) and methionine (M) of the βA spacer OVA were selected in the present disclosure. FAM is a fluorescent dye, 5-carboxyfluorescein, indicating the linkage and modification to FAM, which is commonly used to determine the localization of a drug in a cell. | | | |

The specific operation steps are as follows:
(1) Solid-phase synthesis of peptides: 500 mg Rink amide MBHA resin was weighed in a 10 mL peptide tube, dichloromethane (DCM) was added, and nitrogen was bubbled for 30 min. 50% (v/v) solution of morpholine in N,N-dimethylformamide (DMF) was added and nitrogen was bubbled for 30 min to remove the Fmoc protecting group from the resin. After the resin was washed alternately for 6 times with DMF and DCM, the above steps were repeated twice to remove Fmoc. After alternately washing the resin 6 times with DMF and DCM, DMF solution containing amino acid (5eq, 0.4 M, DMF solvent), 6-chlorobenzotriazole-1,1,3,3-tetramethyluronium hexafluorophosphate (HCTU) (5eq, 0.38 M, DMF solvent) and N,N-diisopropylethylamine (DIPEA) (10eq) was added to the resin by blowing for 2 h. After the reaction solution was removed and the resin was washed six times alternately with DMF and DCM, the Fmoc was removed in the same manner as described above, and then the next amino acid was added until all amino acid connections were complete.
(2) Peptide terminal amidation treatment: N-terminal amino groups were generally acetylated in order to improve the stability of the peptide. The specific steps were as follows: After the final amino acid was attached, the Fmoc group is removed according to the method of step (1), followed by addition of 2.5 mL of amidation reagent (containing: acetic anhydride: DIPEA: DMF (v/v/v) =1:3:16), and after 1 h of nitrogen blowing, the resin was washed 10 times alternately with DMF and DCM. The above procedure was repeated again to complete amidation protection of the N-terminal NH₂.
(3) Cleavage of the peptide: The peptide was cleaved from the resin with a cleavage solution mixed with trifluoroacetic acid (TFA), triisopropylsilane (TIPS) and water (TFA: TIPS: water (v:v:v) =95:2.5:2.5), and the cleavage solution was removed by nitrogen purging, followed by precipitation of the peptide with glacial ether to complete the preliminary purification of the peptide to give the crude product of the M-CP, FAM-OVA, OVA or Adpgk peptide.
(4) The preliminary purified samples were isolated and purified using a C18 high performance liquid chromatography column and identified by LC-MS. The resultant was lyophilized by a lyophilization machine to obtain the peptide pure product with the purity of more than 95%.

### Example 2

### Closure, Isolation and Purification Steps of M-CP Peptide

The HPLC purified M-CP peptide solid was dissolved with a mixing solution containing formic acid, acetonitrile and water (formic acid: acetonitrile: water (v:v:v) =1:5:4) and 10eq of o-dibromobenzyl was added. After the reaction was carried out on a shaker overnight, the reaction mixture was purified by HPLC again and lyophilized to give the closed-loop product of M-CP (Fig. 1a). The introduction of o-dibromobenzyl added two positively charged "sulfonium" centers to the original linear peptide with two methionines, resulting in an increase in the overall positively charged nature of the peptide, which provided a stronger electrostatic driving force for nucleic acid-induced self-assembly. Moreover, the central site of the active "sulfonium" can be reduced by a reducing substance such as intracellular GSH, thereby releasing the internal drug. Rapid *in vitro* self-assembly and *in vivo* reversible reduction release of the closed-loop peptide can be realized through the simple closure step. Figs. 1b and 1c showed that two methionines M in the M-CP peptide were immobilized after addition of o-dibromobenzyl, at which time the molecular weight of the peptide was increased from the original 1551.8 Da to 1655.9 Da. The MS identification results were consistent with the theoretical molecular weight, which indicated that the closed-loop peptide was successfully obtained.

### Example 3

### Preparation, Separation and Purification of OVA with Sulfonium Center

In order to covalently conjugate the tumor antigen peptide with a nucleic immune adjuvant, a methionine was added to the terminus of the OVA peptide. An alkynyl group with a sulfonium center was introduced at this position under acidic conditions. At this time, a highly active alkynyl group can be added to a sulfhydryl group at the end of the nucleic acid under the driving of a positively charged "sulfonium" center to realize covalent conjugation of the peptide and the nucleic acid. The "sulfonium" center site in the conjugate may provide an active site for reversible cleavage of the reductant (e.g., GSH or mercaptopyridine, etc.) to achieve an efficient conjugation *in vitro* and *in vivo* reversible release of the antigen peptide-adjuvant.
(1) The OVA peptide solid was dissolved in a mixing solution containing formic acid, acetonitrile and water (formic acid: acetonitrile: water (v:v:v) =1:5:4), and 10eq of tribromopropyne was added and the reaction was carried out overnight on a shaker to introduce an alkynyl group with a "sulfonium" center at the methionine position of the peptide (Fig. 2a).
(2) The reacted samples were isolated and purified using a C18 high performance liquid chromatography column and identified by LC-MS. Fig. 2b showed that the molecular weight of the FAM-OVA peptide after introduction of the "sulfonium" center is 1603.8 Da, which was consistent with theoretical data. (Fig. 2b is a schematic structural diagram and mass spectrometric identification of FAM-OVA peptide).

### Example 4

### Preparation, Isolation and Purification of CpG-OVA Covalent Conjugates

**Table 2. DNA Sequence Information for Covalent Conjugation to OVA Peptides**

| DNA | Sequence (5'→3') | Length (nt) | |
|---|---|---|---|
| CpG | HS-TCCATGACGTTCCTGACGTT | 20 | SEQ ID NO 6 |

| | | | |
|---|---|---|---|
| Note: HS represents a C6 thiol modification at the 5' end. | | | |

(1) The ordered sulfhydryl-terminated DNA may partially dimerize during transport or due to temperature effects, which may result in reduced covalent conjugating efficiency of the nucleic acid peptide. Therefore, prior to ligation, pre-treatment of the ordered DNA is first required to open disulfide bonds formed by DNA self-aggregation. The specific procedure was as follows: 200eq of TCEP was added to an aqueous solution of 100 µM 50 µL in DNA, and after reaction at room temperature for 4 h, 0.1eq of sodium acetate (pH 5.2, 3 M) and 2.5eq of glacial ethanol were added. After centrifugation at 12000 rpm for 30 min, the supernatant was removed and excess TCEP was removed. 200 µL of glacial ethanol was then added to wash once and the supernatant was removed after 30 min of 12000 rpm centrifugation. After the precipitate was dried on ice for 10 min, it was dissolved in ultrapure water and quantified with Nanodrop.
(2) According to the protocol shown in Fig. 2a, 2eq to 10eq of FAM-OVA peptide was added to the CpG system, and after the reaction was carried out in 10 mM (NH₄)₂CO₃ solution at 4°C overnight, the reaction product was isolated and purified by a C18 high performance liquid chromatography column. The ligation results were detected by PAGE gel electrophoresis. The results of Fig. 2c showed that a new product with a band position lag occurred in the lane after addition of FAM-OVA to the CpG. Further analysis by the FAM fluorescence channel revealed that the product in the CpG-OVA lane differed from FAM-OVA, which demonstrated that the OVA peptide with sulfonium center can be covalently conjugated to the sulfhydryl-terminated CpG.
(3) To further determine the covalent conjugation of CpG to OVA, the final conjugated product was verified by LC-MS and the molecular weight of the DNA-peptide covalently conjugated product was determined to be 7858 Da (Fig. 3b). The molecular weight was equal to the sum of the molecular weight of CpG (6255 Da, Fig. 3a) and the molecular weight of FAM-OVA peptide (1603 Da, Fig. 2b).

### Example 5

### Reverse Reduction of CpG-OVA Covalently Conjugated Product

In order to ascertain that the CpG-OVA covalent conjugate prepared by the present disclosure can be re-reduced and cleaved, the OVA peptide and the immunoadjuvant CpG can be released, and the efficient *in vitro* assembly and *in vivo* reversible release of the nanocomposite are possible, this example demonstrated the reduction of CpG-OVA covalent conjugates by glutathione GSH. 100eq of GSH reducing agent was added to CpG-OVA covalently conjugated product at a final concentration of 40 µM and the reaction was carried out at 37°C in PBS buffer for 0 h, 4 h, 8 h, 12 h, 24 h, 36 h, 48 h, 72 h. The reduction of the covalently conjugated product was analyzed by 10% PAGE gel electrophoresis. The results in Fig. 4 showed that the bands of the initially conjugated product gradually darkened as the reaction time was extended while the amount of CpG substrate gradually increased. The covalent conjugate was determined to be reductively cleavable by a reducing agent.

### Example 6

Rapid Self-assembly of M-CP Closed-loop Peptide Induced by CpG-OVA
(1) Two positively charged "sulfonium" centers were further introduced to the linear M-CP peptides with two methionines after closure, which provided sufficient electrostatic interaction sites for the preparation of nanocomposites. The negatively charged nucleic acid immunoadjuvant CpG in the present disclosure acted as a trigger for self-assembly of a peptide to induce rapid assembly of a positively charged M-CP closed-loop peptide (Fig. 5a, nanocomposite abbreviated as MCN-NPs). It was worth mentioning that the active "sulfonium" centers in both the closed-loop peptide and the CpG-OVA covalent conjugate may both provide sites of action for the reduction of intracellular GSH and the disintegration of nanocomposites. The *in vitro* efficient, rapid self-assembly and *in vivo* reversible reduction release of the nanocomposites was finally achieved. The results in Fig. 5b showed that the CpG-OVA band disappeared as the concentration of the M-CP closed-loop peptide increased, and complete encapsulation of the CpG-OVA was achieved at the 100eq addition. In addition to the aqueous environment, self-assembly of the composites can also be achieved at 10 min in opti-MEM cell culture medium and cell culture medium containing 10% serum (Figs. 5c and 5d).
(2) The microscopic parameters of MCN-NPs were characterized by dynamic nanoparticle particle size meter DLS. The results in Fig. 6a showed that the nanocomposite has a particle size of 54.4±0.9 nm and a PDI index of 0.19±0.02 at CpG-OVA:M-CP= 1:300 (PDI index was used to evaluate the homogeneity of the nanocomposite, the numerical range was 0 to 1, and the smaller the numerical range indicated that the higher the homogeneity of the particles, generally less than 0.3). The results in Fig. 6b showed that with the addition of M-CP, the charge of the composite system gradually changed from the original negative charge to the positive charge, again confirming that the negatively charged nucleic acid induced the self-assembly of the positively charged M-CP closed-loop peptide.
(3) Furthermore, the micromorphology of MCN-NPs was observed by transmission electron microscopy TEM in the present disclosure. The electron microscope results in Fig. 6c show that the nanocomposite was a uniform sphere with a particle size consistent with the results of DLS.

### Example 7

### Stability Evaluation of MCN-NPs

Due to the stability of the peptide, it was difficult to effectively activate the host immune system when the tumor antigen peptide was administered alone, resulting in poor clinical trials of new tumor antigens. Encapsulation of antigen peptides by nanocomposites played an important role in enhancing the stability and efficacy of antigen peptides. The stability of MCN-NPs in aqueous phase and culture medium containing 10% serum RPMI 1640 cells was evaluated in the present disclosure. After 24 h incubation at 4°C, DLS analysis showed that the particle size and potential of MCN-NPs did not increase significantly (Fig. 7), indicating that the nanocomposite prepared according to the present disclosure had high stability.

### Example 8

### Security Evaluation of MCN-NPs

The safety and compatibility of the tumor antigen delivery system was a prerequisite for its clinical application and promotion. To this end, the present disclosure evaluated the biological safety of MCN-NPs using classical MTT methods and hemolysis assays.
(1) For the MTT assay, RAW264.7 cells were plated in advance in 96-well plates, and the maximum dose of 1 µM of CpG-OVA (wherein the M-CP dose was normalized to the OVA dose) was administered according to CpG-OVA:M-CP=1:300, and then diluted sequentially in a gradient manner. After 24 hours of administration, 20 µL of MTT (3-(4,5-dimethylthiazol-2)-2,5-diphenyltetrazolium bromide) was added to each well, and was incubated at 37°C for an additional 4 hours. After careful aspiration of the cell supernatant, 100 µL of methadone crystals produced by lysis of viable cells dissolved with DMSO was added into each well. The absorbance was measured at 490 nm for each well using a full-function enzyme calibration. The growth inhibition rate of cells was calculated and the cytotoxicity of MCN-NPs was evaluated using cells without drug as a control group. The results in Fig. 8 showed that the cells in the MCN-NPs group still had nearly 100% cell survival at the maximum administered dose, and that MCN-NPs had a weak pro-growth effect at low drug concentrations, indicating that MCN-NPs had no significant cytotoxicity.
(2) Furthermore, the biological safety of MCN-NPs was evaluated by cell hemolysis assay. Fresh mouse whole blood was removed by centrifugation at 1200 rpm for 5 min and the cells were washed with PBS until the supernatant was colorless. Subsequently, MCN-NPs with different concentrations (maximum dose of 1 µM, calculated as OVA concentration) were mixed with 10⁵ red blood cells. The mixture was incubated at 37°C for 1 h. Subsequently, the supernatant was centrifuged at 1200 rpm for 5 min, and 100 µL of supernatant was taken into a 96-well plate. The absorbance of the supernatant was measured at 570 nm using a full-function enzyme calibration to evaluate heme dissolution. The results in Fig. 9a showed that the color of the cell supernatant in the MCN-NPs incubation group was similar to that in the PBS control group. Further determination of the absorbance of the supernatant at 570 nm revealed that heme dissolution associated with red blood cell membrane fragmentation was almost undetectable in the supernatant upon administration of the maximum MCN-NPs (Fig. 9b). The constructed antigen delivery system of the present disclosure had also been shown to have good biological safety, which provided an important guarantee for the subsequent use of the nano-system in the neoplastic antigen immunotherapy.

### Example 9

### Evaluation of MCN-NPs Uptake by Macrophages RAW 264.7

The uptake of tumor antigens by immune cells was a key step in activating the immune system. Flow cytometry and laser confocal microscopy (Confocal) were used in the present disclosure to evaluate MCN-NPs uptake by immune cells. Firstly, RAW264.7 cells were treated with OVA peptide with green fluorescent tag (FAM group), CpG-OVA covalent conjugate and MCN-NPs (1 µM as OVA), respectively. The uptake of MCN-NPs into RAW264.7 cells was analyzed by flow cytometry and laser confocal microscopy after 4 hours.
(1) For flow analysis: Cells treated for 4 h were washed three times with PBS, then digested with 0.25% trypsin for 1 min, and 10% RPMI1640 was added to neutralize the trypsin. The centrifugation was carried out at 1000 rpm and 4°C to remove supernatant. The cells were washed with PBS in accordance with resuspension-centrifugation-resuspension, followed by on-machine detection. The flow results in Fig. 10a showed that the MCN-NPs experimental group had the strongest fluorescence shift compared with the OVA group, confirming that the M-CP nanocomposite did significantly improve the cell membrane penetration capacity of the OVA peptide.
(2) For the confocal experiment: Cells were spread on cell climbing pieces 12 h in advance, the climbing pieces were carefully washed with PBS after dosing treatment, and then fixed cells were treated with 4% paraformaldehyde for 15 min. The residual formaldehyde was washed twice with PBS. Finally, the pieces were sealed with a sealing tablet containing DAPI. After drying at room temperature in the dark, the green fluorescence intensity and localization in RAW264.7 cells in different experimental groups were observed with 488 nm laser. The Confocal results in Fig. 10b also showed that the cells in the MCN-NPs treated group had the strongest green fluorescence of FAM, which was consistent with the flow detection results in Fig. 10a. Furthermore, M-CP did significantly increase the uptake of OVA antigen by macrophages.

### Example 10

### Evaluation of MCN-NPs Uptake by Antigen-presenting Cells DC2.4

As important antigen presenting cells, the uptake of antigen by DC cells directly determined their ability to induce T cell activation. The present disclosure further assessed the uptake of MCN-NPs by the stably culturable antigen presenting cell line DC2.4. Flow cytometry and laser confocal microscopy were also used to analyze the membrane penetration and localization of MCN-NPs after internalization. The flow-through procedure was the same as that in Example 9. For confocal experiments, DC cells shrank severely due to paraformaldehyde fixation, in which the degree of internalization of MCN-NPs was analyzed using live cell imaging techniques. The procedure was as follows: DC2.4 cells were plated in 8-well plates for living cell imaging 12 h in advance, and after 4 h of administration, the culture supernatant was removed, and a mixing solution of PBS containing Hoechst33342 (a nuclear blue fluorescent dye for living cells) and Lysotracker (a lysosomal red fluorescent dye for living cells) was added, and after incubation at 37°C for 30 min, the supernatant was discarded. Careful washing was carried out three times with PBS, followed by addition of 10% RPMI 1640 medium to allow on-machine detection. The flow results in Figs. 11a and 11b showed that in the DC2.4 cell line, the MCN-NPs treated group had the greatest fluorescence shift. Similarly, the Confocal data (Fig. 11c) showed the strongest green fluorescence in DC2.4 cells in the MCN-NPs group. The above data again demonstrated that M-CP was effective in assisting the penetration of antigen peptides.

### Example 11

### Evaluation of MCN-NPs Uptake by Mouse Bone Marrow Source BMDCs

The mature cell lines RAW264.7 and DC2.4 can initially demonstrate the internalization of nanocomposites by immune cells at the cellular level, and whether MCN-NPs had the same effect on DC cells *in vivo* required further experimental verification. In order to further evaluate the *in vivo* immune cell internalization of MCN-NPs, bone marrow DC cells from C57BL/6 mice were extracted according to a mature published method (refer to the extraction and culture procedure of BMDCs, available from abcom, Inc.: https://www.abcam.cn/proteins/bmdc-isolation-protocol) and examined for internalization of MCN-NPs.

The culture procedure for BMDCs was as follows:
C57BL/6 mice of 2 to 4 weeks of age were used and sacrificed with CO₂. The femur and tibia of the mice were harvested under sterile conditions. Both ends of the bone joint were cut with scissors, and the bone marrow was washed with serum-free RPMI medium until the bone cavity became white. After centrifugation at 1500 rpm for 5 min, the supernatant was removed and the cells were resuspended in red blood cell lysate for 3 min before the lysis was terminated in RPMI 1640 medium supplemented with 5 mL containing 10% serum. The supernatant was removed after centrifugation at 1500 rpm for 5 min, followed by washing twice with RPMI 1640. The cells were resuspended with 10%RPMI1640 containing 20 ng/mL GM-CSF and 10 ng/mL IL-4 factors. After counting, the cells were seeded into 6-well plates at a density of 1×10⁶ cells/mL, at which time day 0 of culture was recorded. After 2 days of culture, a 3/4 exchange was performed, followed by exchange of a half volume on day 4, and immature murine BMDCs cells were harvested on day 6. After the plate was laid for 12 h, the treatment of BMDCs was performed. The internalization of MCN-NPs was determined by flow and laser confocal (the procedure was the same as that in Example 10).

The results in Fig. 12 showed that the MCN-NPs uptake by BMDCs was greater at 4 h after administration compared with OVA peptide alone. With the increase of the administration time, the green fluorescence intensity in BMDCs increased first and then decreased, and significant green fluorescence was still observed in the cells of the MCN-NPs-treated group at 48 h (Fig. 13). This result was consistent with the data of DC2.4, and again fully demonstrated that the peptide-based nanocomposites prepared according to the present disclosure can significantly improve the stability of tumor antigen peptides, the ability to penetrate membranes and the effect of intracellular controlled release.

### Example 12

### Evaluation of Immune Activation Ability of MCN-NPs

The secretion of tumor-associated factors and interleukin from immune cells stimulated by drug may reflect the immune system activity to some extent. In the present disclosure, the ability of MCN-NPs to stimulate the activation of the immune system was assessed by the secretion of tumor-associated factors TNF-α and interleukin IL-6, II,12p40 by RAW264.7 cells following administration of a drug with an enzyme-linked immunosorbent assay (ELISA) kit. RAW264.7 cells were treated with CpG, OVA, CpG-OVA, MCN-NPs (at a concentration of 1 µM on an OVA basis) for 48 h, respectively. The cell supernatants were collected. The suspended cells were subsequently removed by centrifugation at 1000g for 20 min. 100 µL of cells supernatant was added to an enzyme-linked immunoassay plate. The secretion of each cytokine by RAW264.7 cells was analyzed according to the instructions of the ELISA kit. The contents of tumor-associated factors TNF-α (Fig. 14a), interleukin IL-6 (Fig. 14b) and IL-12p40 (Fig. 14c) in the cell culture supernatant were calculated by drawing a standard curve from the ELISA results of the standard samples in the kit. The results in Fig. 14 showed that the MCN-NPs nanocomposite significantly increased the amount of secretion of each related factor compared with the administration of OVA or CpG alone, which demonstrated the potential tumor immunotherapy capabilities.

### Example 13

### Evaluation of Ability of MCN-NPs to Activate Immune System In Vivo

Experimental results at the cellular level preliminarily confirmed that the nanoparticles constructed in accordance with the present disclosure had potential anti-tumor immune effects. To further evaluate the effectiveness of the vaccine, the activation of the immune system stimulated by MCN-NPs was assessed at the animal level. In order to determine the enrichment of the nanocomposites in the lymph nodes, Cy5.5 was fluorescently modified at the 5' end of CpG, and then MCN-NPs-Cy5.5 was prepared according to Example 6. After 24 hours of subcutaneous administration to the tail, the mice were treated with CO₂ to obtain the mouse lymph nodes and subjected to *in vitro* tissue fluorescence imaging analysis. The results in Fig. 15a showed that the lymph nodes of MCN-NPs were significantly increased compared with the control and CpG groups. Further imaging analysis revealed that the fluorescence intensity of the lymph nodes in the MCN-NPs group was 3.77-fold higher than that in the CpG group (Fig. 15b). It was shown that the nanocomposites effectively entered the lymph nodes under the action of draining lymph nodes.

To further evaluate whether the nanocomposites entering the lymph nodes were effective in activating the associated immune cells. MCN-NPs nanocomposites (15 nmol/in terms of the amounts of OVA) were dosed every 7 days according to the time points of Fig. 16a, and a mixture of M-CP and CpG (15 nmol/in terms of the amount of CpG) was added once over the 7-day interval. Seven days after the last administration of the MCN-NPs nanocomposites, the lymph node stimulation was again analyzed and found that the inguinal lymph nodes in the MCN-NPs group were the largest and the number of cells was the largest (Figs. 16b, 16c and 16d). The lymph nodes were subsequently milled into single cells in a 70 µM disposable nylon filter using a syringe plunger, 1 mL of PBS was added to resuspend single cells, and after centrifugation at 500g for 5 min at room temperature, the supernatant was removed. 1 mL of PBS was added to resuspend again and 50 µL of cell resuspension was taken and the cell number was counted by flow cytometry. The number of 2×10⁷ cells/tube was divided into three parallel groups. After centrifugation, the cells were resuspended in 100 µL cell flow buffer and 1.0 µL of CD16/CD32 antibody was added. After 10 min incubation at room temperature, CD11c-APC (for labeled DC cells), CD19-PE (for labeled B cells), F4/80-APC (for labeled macrophages) were added, respectively and incubated at room temperature for 20 min. 1 mL of PBS was supplemented. The supernatant was then removed after centrifugation at room temperature for 5 min. PBS was used to wash again. The cells were resuspended in 500 µL cell flow buffer. The fluorescently labeled cells were assessed on the machine. The results in Fig. 17 showed that DC cells (Fig. 17a) and B cells (Fig. 17b) in lymph nodes were significantly activated by MCN-NPs for proliferation. The results in Fig. 17c showed that the proportion of macrophages in the MCN-NPs group did not increase significantly, possibly because macrophages were associated with innate immunity, and the cellular level associated with innate immunity was returned to equilibrium after 7 days of administration.

The changes of T cells in the mouse blood were then continuously detected, and the specific procedure was as follows: Fresh mouse blood was obtained using orbital blood sampling, and heparin sodium was added to prevent coagulation. The blood was then divided into 50 µL, lysed for 10 min by adding 1 mL 1× red blood cell lysate, and after centrifugation at 500g for 5 min at room temperature, the supernatant was removed. Cells were resuspended with 1 mL of cell flow buffer, centrifuged and washed again. The cells were then resuspended in 100 µL of cell flow buffer. T-cell labeling in the blood was performed according to the lymph node staining procedure. CD3e-efluor450, CD4-APC and CD8-FITC were added to the above samples simultaneously to label the CD4+ and CD8+ T-cell populations in the blood. Simultaneous addition of CD3e-efluor450, CD8-FITC and MHC-OVA-APC was used to label OVA-specific T cells in the CD8+ cell population in the blood. The results in Fig. 18 showed that the total population of T cells in the blood of mice (Fig. 18a), the proportion of CD8+ T cells (Fig. 18b) and the proportion of OVA-specific killer T cells (Fig. 18c) increased at day 7 after the end of immunization. Moreover, the ability of MCN-NPs to induce T cells to differentiate into CD8+ killer T cells was enhanced compared with the physically mixed administration of CpG and OVA, with a significant increase in the proportion of OVA-specific CD8+ T cells (Fig. 18d). It was shown that the nanocomposites constructed according to the present disclosure significantly activated the cellular immune system in mice.

### Example 14

### MCN-NPs for Prevention of Tumors

Furthermore, the present disclosure constructed two tumor prevention models for evaluating the prevention of tumor colonization and growth with the nanocomposites. The specific model settings were as follows:
Tumor prevention model:
The control group (0% 1640), the OVA group (15 nmol/mice), the CpG+OVA group (CpG 15 nmol/mice, OVA 15 nmol/mice), the CpG-OVA covalent conjugate (15 nmol/mice), the MCN-NPs (CpG-OVA 15 nmol/mice, M-CP 150 nmol/mice) and the M-CP group (150 nmol/mice) were set. According to the protocol of Fig. 19a, three doses were administered every 7 days. The MCN-NPs group was supplemented with a mixture of M-CP and CpG once at an interval of 2 days after each dose (15 nmol/, M-CP 150 nmol/, calculated as the amount of CpG). 1×10⁶ B16-OVA cells/mouse were inoculated subcutaneously 10 days after the end of the last immunization. Tumor size was measured and body weight changes were counted after 6 days. Experiments were terminated when the tumor grew to 1500 mm³ which was the tumor statistical endpoint. Subsequently, the life cycle statistics were carried out.

The results in Figs. 19b to 19d showed that tumors in mice in the nanocomposite group were significantly inhibited with an inhibition rate of 100%, and the life cycle of the mice in this group was significantly increased (Fig. 19e). No significant body weight changes were detected throughout the study (Fig. 20), indicating that the nanocomposites had good biological safety and played a positive role in preventing tumorigenesis.

Lung Metastasis Model:
Furthermore, a lung metastasis model was selected to evaluate the prevention of lung colonization of melanoma with nanocomposites (Fig. 21a). The administration was similar to that of the tumor model, except that 5×10⁵ B16-OVA cells per mouse were injected via tail vein 7 days after the end of the last administration. The mice were dissected on day 20 after injection, the lungs of the mice were removed, and the lungs were washed with Fekete's eluent (70 mL of alcohol, 10 mL of formaldehyde, 5 mL of glacial acetic acid, 15 mL of water). After decoloration, lung tumor nodules were counted. Finally, all tissues were sent to Univ company for tissue section and H&E staining analysis. The results in Figs. 21b and 21c showed that the MCN-NPs group had the least number of black nodules in the lung. The H&E staining analysis of the lung tissue revealed that the control group was able to detect significant lung tissue substantiation and melanoma cell infestation. Similarly, lung cells infected with melanoma cells were also found in the OVA, CpG+OVA and CpG-OVA covalent conjugate groups. The absence of this phenomenon in the MCN-NPs nanocomposite group indicated that the nanocomposite was effective in preventing lung metastasis of melanoma. No significant pathological and physiological structural changes were detected in H&E staining of important tissues other than the lungs (Fig. 22) compared with the control group, which also predicted that the nanocomposites constructed in this study had good safety.

### Example 15

### MCN-NPs for Treatment of Tumors

The present disclosure then constructed a tumor treatment model for evaluating the efficacy of nanocomposites in the treatment of melanoma. According to the time points of Fig. 23a, 1×10⁶ B16-OVA cells per mouse were inoculated subcutaneously on day 0. After 6 days, the tumor size was measured and the body weight change of mice was counted and the first administration was performed. A total of three doses were then administered every 7 days. The MCN-NPs group was supplemented with a mixture of M-CP and CpG once at an interval of 2 days after each dose (15 nmol/, M-CP 150 nmol/, calculated as the amount of CpG). The tumor volume growth in mice was counted continuously over the period. The tumor growth to 1500 mm³ was the tumor statistical endpoint. Subsequently, the life cycle analysis was carried out. The results in Fig. 23b and 23e showed that tumor growth was slowest in the MCN-NPs group, and tumors in three mice were eliminated 27 days after inoculation, achieving 75% tumor treatment. The life cycle of this group of mice was also significantly increased (Fig. 23c), again demonstrating that the nanoparticle vaccines constructed according to the present disclosure had a significant tumor therapeutic effect. Similarly, no significant body weight loss in mice was detected throughout the experiment, indicating the safety of the nanocomposites (Fig. 23d).

### Example 16

### MCN-NPs for Treatment of Other Tumors

To verify the compatibility and general applicability of the delivery system of the present disclosure, the model antigen peptide OVA was replaced with the neoplastic antigen peptide Adpgk (Ac-ELASMTNMELMSS, SEQ ID NO 4) of mouse colorectal cancer MC-38. The covalent conjugate of CpG-Adpgk was prepared according to the protocol of Examples 1 to 4. The covalent conjugate was mixed with the M-CP peptide to obtain a nanocomposite containing the novel antigen peptide of Adpgk (designated as MCN-NPs_{(Adpgk)}). The present disclosure further constructed an anti-tumor model of MC-38 as follows: 1×10⁶ MC-38 cells per mouse were inoculated subcutaneously on day 0. After 7 days, tumor size was measured and the body weight changes were counted and the first administration was performed. A total of three doses were then administered every 7 days. The control group (0% 1640), the Adpgk group (15 nmol/mouse), the CpG+Adpgk group (CpG 15 nmol/mouse, Adpgk 15 nmol/mouse), the MCN-NPs_{(Adpgk)} (CpG-Adpgk 15 nmol/mouse, M-CP 150 nmol/mouse) and the M-CP group (150 nmol/mouse) were provided. Tumor volume growth in mice was continuously counted during the period. The statistical data were included until the tumor grew to 1000 mm³ as the tumor statistical endpoint. Tumors of each group of mice were obtained at day 34 post-seed tumor for data statistical analysis. From the MC-38 tumor growth curves of Figs. 24a (statistical data) and 24d (individual data), it can be seen that the tumor volume growth was the slowest in the MCN-NPs treated group, followed by the M-CP treated group, which was consistent with the results of Fig. 23b. It was shown that the nanoparticle vaccines constructed by the present disclosure had a significant and tumor-specific tumor treatment effect. As can be seen more intuitively from the tumor weights and comparison figures of Figs. 24b, 24c and 24e, tumors in 3 mice in the MCN-NPs_{(Adpgk)} treatment group were eliminated at 34 days, achieving a tumor treatment rate of 60%. The effectiveness and general applicability of the constructed nanoparticle vaccines of the present disclosure were demonstrated again. Similarly, no significant body weight loss in mice was detected throughout the experiment (Fig.25), indicating the safety of the nanocomposites.

In the present disclosure, relational terms, such as first and second and the like, are used solely to distinguish one entity or operation from another entity or operation without necessarily requiring or implying any such actual relationship or order between such entities or operations.

From the foregoing it will be appreciated that, although specific embodiments of the present disclosure have been described herein for illustrative purposes, various modifications or improvements may be made by those skilled in the art without departing from the spirit and scope of the present disclosure. Such variations or modifications are intended to fall within the scope of the claims appended hereto.

## Claims

1. A composition comprising:
a closed-loop peptide comprising at least two methionines and at least two sulfonium centers; and
a conjugate formed by a negatively charged immunoadjuvant and a peptide.

2. The composition of claim 1, wherein the negatively charged immunoadjuvant is selected from the group consisting of a nucleic acid immunoadjuvant, an activator of an immune pathway and an adjuvant having an effect of blocking an immunosuppressive pathway, preferably CpG₁₈₂₆, ploy(I:C) and siRNA/shRNA for silencing an immunosuppressive pathway or activating an immune pathway.

3. The composition of claim 1 or 2, wherein the negatively charged immunoadjuvant comprises at least 20 nucleotides.

4. The composition of any one of claims 1 to 3, wherein the negatively charged immunoadjuvant comprises a sequence of SEQ ID NO 6.

5. The composition of any one of claims 1 to 4, wherein the peptide is an antigen peptide, preferably an antigen peptide of melanoma B16-OVA (OVA₂₅₇₋₂₆₄: Ac-SIINFEKL) and a neoantigen peptide of MC-38 tumor cells (Adpgk: Ac-ELASMTNMELMSS).

6. The composition of any one of claims 1 to 5, wherein the peptide comprises at least 8 amino acids.

7. The composition of any one of claims 1 to 6, wherein the peptide comprises a sequence of SEQ ID NO 2, a sequence of SEQ ID NO 3, or a sequence of SEQ ID NO 4.

8. The composition of any one of claims 1 to 7, wherein the closed-loop peptide comprises at least 9 amino acids.

9. The composition of any one of claims 1 to 8, wherein the closed-loop peptide comprises a sequence of SEQ ID NO 1.

10. The composition of any one of claims 1 to 9, wherein the conjugate is a reversible covalent conjugate.

11. The composition of any one of claims 1 to 10, wherein the composition has a particle size of 50 nm to 200 nm.

12. A process for preparing the composition of claim 1, comprising:
dissolving a linear peptide comprising at least two methionines in a weakly acidic polar solution and adding a closed-loop reagent to obtain a closed-loop peptide;
mixing an immunoadjuvant with a sulfonium peptide under basic conditions to obtain a conjugate by an addition reaction; and
mixing the conjugate with the closed-loop peptide to obtain the composition.

13. The process of claim 12, wherein the immunoadjuvant is a nucleic acid adjuvant, preferably an immunoadjuvant having a sulfhydryl terminus, more preferably a nucleic acid immunoadjuvant comprising a sequence of SEQ ID NO 6.

14. The process of claim 12 or 13, wherein the sulfonium peptide is selected from the group consisting of a sulfonium peptide having an alkynyl terminus and a sulfonium peptide having a vinyl terminus, preferably selected from the group consisting of a peptide comprising a sequence of SEQ ID NO 2, a peptide comprising a sequence of SEQ ID NO 3 and a peptide comprising a sequence of SEQ ID NO 4.

15. The process of any one of claims 12 to 14, wherein the molar ratio of the immunoadjuvant to the sulfonium peptide is from 1:2 to 1:10.

16. The process of any one of claims 12 to 15, wherein the molar ratio of the immunoadjuvant to the closed-loop peptide is 1:50 to 1:400.

17. The process of any one of claims 12 to 16, wherein the conjugate is self-assembled with the closed-loop peptide to obtain the composition.

18. The process of any one of claims 12 to 17, wherein the immunoadjuvant is mixed with a sulfonium peptide under basic conditions at 4 to 28°C to obtain a conjugate by an addition reaction.

19. The process of any one of claims 12 to 18, wherein the linear peptide comprising at least two methionines comprises a sequence of SEQ ID NO 1.

20. A method for treating or preventing a tumor, comprising administering to a subject in need thereof a therapeutically or prophylactically effective amount of the composition of any one of claims 1 to 11.

21. The method of claim 20, wherein the tumor is selected from the group consisting of melanoma and colon cancer.

22. The method of claim 20 or 21, wherein the subject is a mammal, preferably a human.

23. The method of any one of claims 20 to 22, further comprising administering to the subject another agent for preventing or treating a tumor.

24. The method of claim 23, wherein the agent is selected from the group consisting of a chemotherapeutic drug, a biological drug or any combination thereof, preferably a small molecule chemotherapeutic drug, a peptide drug, an antibody drug or any combination thereof, more preferably paclitaxel, PD-1 monoclonal antibody, PD-L1 monoclonal antibody, a biological drug having a targeted therapeutic effect or any combination thereof.

25. A method for activating immunity, comprising administering to a subject in need thereof an effective amount of the composition of any one of claims 1 to 11.

26. The method of claim 25, wherein the immunity is tumor immunity.

27. The method of claim 25 or 26, wherein the immunity is cellular immunization.

28. A nanoparticle comprising:
a closed-loop peptide comprising at least two methionines and at least two sulfonium centers; and
a conjugate formed by a negatively charged immunoadjuvant and a peptide,
wherein the closed-loop peptide is self-assembled with the conjugate to form the nanoparticle.
